# EUROPEAN PATENT SPECIFICATION

(11) **EP 1 131 070 B1**
(45) Date of publication and mention of the grant of the patent: **03.09.2008**
(21) Application number: 99964483.4
(22) Date of filing: 08.11.1999
(51) Int. Cl.: A61K 31/353, A61K 31/4439, A61K 9/32, A61K 9/52, A61P 3/10

(54) **PHARMACEUTICAL COMPOSITION FOR MODIFIED RELEASE OF AN INSULIN SENSITISER AND METFORMIN**
ARZNEIMITTEL ZUR GESTEUERTEN FREISETZUNG EINES INSULIN SENSIBILISATORS UND METFORMIN
COMPOSITION PHARMACEUTIQUE A LIBERATION MODIFIEE D'UN AGENT DE SENSIBILISATION A L'INSULINE, ET DE METFORMINE

(30) Priority: 12.11.1998 GB 9824866; 12.11.1998 GB 9824867; 12.11.1998 GB 9824869; 25.05.1999 GB 9912193; 25.05.1999 GB 9912190; 25.05.1999 GB 9912191
(43) Date of publication of application: 12.09.2001
(60) Divisional application: 07105992.7
(73) Proprietor: SMITHKLINE BEECHAM PLC, Brentford, Middlesex TW8 9GS (GB)
(72) Inventor: LEWIS, Karen SmithKline Beecham Pharmaceuticals, Harlow Essex CM19 5AW (GB); LILLIOTT, Nicola, Jayne SmithKline Beecham Pharma., Harlow Essex CM19 5AW (GB); MACKENZIE, Donald Colin SmithKline Beecham Pharma., Harlow Essex CM19 5AW (GB); RE, Vincenzo SmithKline Beecham Pharmaceuticals, Harlow Essex CM19 5AW (GB)
(74) Representative: Rutter, Keith
(86) International application number: PCT/EP1999/008704
(87) International publication number: WO 2000/028989

(56) References cited:
- EP-A- 0 861 666
- WO-A-98/11884
- WO-A-98/42340
- WO-A-99/03477
- DE-A- 4 432 757
- US-A- 5 502 078

## Description

This invention relates to a novel composition, in particular to a modified release composition and its use in medicine, especially its use for the treatment of diabetes mellitus, preferably Type 2 diabetes, and conditions associated with diabetes mellitus.

Alpha glucosidase inhibitor antihyperglycaemic agents (or alpha glucosidase inhibitors) and biguanide antihyperglycaemic agents (or biguanides) are commonly used in the treatment of Type 2 diabetes. Acarbose, voglibose, emiglitate and miglitol are examples of alpha glucosidase inhibitors. 1,1 - Dimethylbiguanidine (or metformin) is a particular example of a biguanide.

Insulin secretagogues are compounds that promote increased secretion of insulin by the pancreatic beta cells. The sulphonylureas are well known examples of insulin secretagogues. The sulphonylureas act as hypoglycaemic agents and are used in the treatment of Type 2 diabetes. Examples of sulphonylureas include glibenclamide (or glyburide), glipizide, gliclazide, glimepiride, tolazamide and tolbutamide.

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter'Compound (I)'). WO94/0565 discloses certain salts of Compound (I) including the maleate salt at example 1 thereof.

Compound (I) is an example of a class of anti-hyperglycaemic agents known as 'insulin sensitisers'. In particular Compound (I) is a thiazolidinedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

It is now indicated that certain modified release pharmaceutical compositions allow administration of a single daily dose of Compound (I) and another antidiabetic agent, such as an alpha glucosidase inhibitor, a biguanide or an insulin secretagogue, to provide an advantageous delivery of drug for maintaining effective glycaemic control with no observed adverse side effects. Such modified release is therefore considered to be particularly useful for the delivery of insulin sensitisers in combination with other antidiabetic agents for the treatment of diabetes mellitus, especially Type 2 diabetes and conditions associated with diabetes mellitus.

Accordingly, the invention provides a pharmaceutical composition suitable for once daily administration, which composition comprises:
(a) 2 mg to 12 mg of 5-[4-[2-(N-methyl-N-(2-pyridyl)amino)ethoxy]benzyl]thiazolidine-2,4-dione, optionally in the form of a pharmaceutically acceptable salt or solvate thereof;
(b) 100 mg to 3000 mg of metformin or a pharmaceutically acceptable salt thereof; and
(c) a pharmaceutically acceptable carrier;
wherein the composition is arranged to provide sustained release of 5-[4-[2-(N-methyl-N-(2-pyridyl)amino)ethoxy]benzyl]thiazolidine-2,4-dione and metformin, said sustained release being provided by a sustained release matrix selected from disintegrating, non-disintegrating and eroding matrices.

In a further aspect the invention provides a process for preparing a pharmaceutical composition according to claim 1, which process comprises formulating [2-(N-methyl-N-(2-pyridyl)amino)ethoxy]benzyl]thiazolidine-2,4-dione or a pharmaceutically acceptable derivative thereof, metformin or a pharmaceutically acceptable salt thereof and the pharmaceutically acceptable carrier so as to enable sustained release of 5-[4-[2-(N-methyl-N-(2-pyridyl)amino)ethoxy]benzyl]thiazolidine-2,4-dione and metformin.

The sustained release may provide effective release of active agents over a time period of up to 26 hours, typically in the range of 4 to 24 hours.

Sustained release is typically provided by use of a sustained release matrix, usually in tablet form, such as disintegrating, non-disintegrating or eroding matrices.

Sustained release is suitably obtained by use of a non disintegrating matrix tablet formulation, for example by incorporating Eudragit RS into the tablet. Alternative non disintegrating matrix tablet formulations are provided by incorporating methacrylates, cellulose acetates, hydroxypropyl methylcellulose phthalate, in particular Eudragit L and RL, Carbopol 971P, HPMCP-HP-55S into the tablet.

Sustained release is further obtained by use of a disintegrating matrix tablet formulation, for example by incorporating methacrylates, methylcellulose, in particular Eudragit L, Methocel K4M into the tablet.

Sustained release can also be achieved by using a semi-permeable membrane coated tablet for example by applying methacrylates, ethylcellulose, cellulose acetate, in particular Eudragit RS, Surelease to the tablet.

Sustained release can also be achieved by using a multi layer tablet, where each active ingredient is formulated together or as a separate layer, for example as a matrix tablet, with the other layers providing further control for sustained release of either one or both active agents.

Suitable dosages, preferably unit dosages, of Compound (I) and metformin, include the known permissible doses for these compounds as described or referred to in reference texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or the above mentioned publications.

The dosages of each particular active agent in any given composition can as required vary within a range of doses known to be required in respect of accepted dosage regimens for that compound. Dosages of each active agent can also be adapted as required to take into account advantageous effects of combining the agents as mentioned herein.

Suitably the composition comprises 2, 3, 4, 5, 6, 7, 8, 9, 10, 11 or 12 mg of Compound (I).

Particularly, the composition comprises 2 to 4 , 4 to 8 or 8 to 12 mg of Compound (I).

Particularly, the composition comprises 2 to 4mg of Compound (I).

Particularly, the composition comprises 4 to 8mg of Compound (I).

Particularly, the composition comprises 8 to 12 mg of Compound (I).

Preferably, the composition comprises 2 mg of Compound (I).

Preferably, the composition comprises 4 mg of Compound (I).

Preferably, the composition comprises 8 mg of Compound (I).

A suitable dosage of metformin is between 100 to 3000mg, for example 250, 500mg, 850mg or 1000mg.

Compound (I) may exist in one of several tautomeric forms, all of which are encompassed by the invention as individual tautomeric forms or as mixtures thereof. The compounds mentioned herein may contain one or more chiral carbon atoms and hence can exist in two or more stereoisomeric forms, all of which are encompassed by the invention either as individual isomers or as mixtures of isomers, including racemates.

It will be understood that Compound (I) and metformin are in a pharmaceutically acceptable form, including pharmaceutically acceptable derivatives such as pharmaceutically acceptable salts, esters and solvates thereof. In certain instances herein the names used for the antidiabetic agent may relate to a particular pharmaceutical form of the relevant active agent: It will be understood that all pharmaceutically acceptable forms of the active agents per se are encompassed by this invention.

Suitable pharmaceutically acceptable forms of Compound (I)and metformin depend upon the particular agent used but include are known pharmaceutically acceptable forms. Such derivatives are found or are referred to in standard reference texts such as the British and US Pharmacopoeias, Remington's Pharmaceutical Sciences (Mack Publishing Co.), The Extra Pharmacopoeia (London, The Pharmaceutical Press) (for example see the 31 st Edition page 341 and pages cited therein) and the above mentioned publications. For example, a particular form of metformin is metformin hydrochloride.

Suitable pharmaceutically acceptable forms of Compound (I) include those described in EP 0306228 and WO94/05659, especially pharmaceutically acceptable salted or solvated forms. A preferred pharmaceutically acceptable salt form of Compound (I) is a maleate. A preferred pharmaceutically acceptable solvated form of Compound (I) is a hydrate.

Compound (I) or, a pharmaceutically acceptable salt thereof, or a pharmaceutically acceptable solvate thereof, may be prepared using known methods, for example those disclosed in EP 0306228 and WO94/05659.

When used herein the term 'conditions associated with diabetes' includes those conditions associated with the pre-diabetic state, conditions associated with diabetes mellitus itself and complications associated with diabetes mellitus.

When used herein the term 'conditions associated with the pre-diabetic state' includes conditions such as insulin resistance, including hereditary insulin resistance, impaired glucose tolerance and hyperinsulinaemia.

'Conditions associated with diabetes mellitus itself' include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type 2 diabetes, neuropathy and retinopathy.

Renal diseases associated with Type 2 diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term 'pharmaceutically acceptable' embraces a veterinarily acceptable compound.

For the avoidance of doubt, unless other wise stated, when reference is made herein to scalar amounts, including mg amounts, of the active compound such as Compound (I), in a pharmaceutically acceptable form, the scalar amount referred to is made in respect of the active compound *per se*: For example 2 mg of Compound (I) in the form of the maleate salt is that amount of maleate salt which provides 2 mg of Compound (I).

Diabetes mellitus is preferably Type 2 diabetes.

Glycaemic control may be characterised using conventional methods, for example by measurement of a typically used index of glycaemic control such as fasting plasma glucose or glycosylated haemoglobin (Hb Alc). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

In a preferred aspect, the dosage level of each of the active agents when used in accordance with the treatment of the invention will be less than would have been required from a purely additive effect upon glycaemic control.

There is also an indication that the treatment of the invention will effect an improvement, relative to the non-modified release of the individual agents, in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

Usually the compositions are adapted for oral administration. However, they may be adapted for other modes of administration, for example parenteral administration, sublingual or transdermal administration.

The compositions are formulated to provide the modified release of active agents according to the appropriate methods required, for example those disclosed in Sustained and Controlled Release Drug Delivery Systems, Editor Joe R Robinson, Volume 7, published by Marcel Dekker under the title Drugs and the Pharmaceutical Sciences, Controlled Drug Delivery, 2nd Edition' edited by Joe Robinson and Vince Lee, Marcel Dekker, 1987 and 'Drug Delivery to the Gastrointestinal Tract' Editors: J G Hardy, S S. Davis and C G Wilson also with reference to texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) and Harry's Cosmeticology (Leonard Hill Books).

The tablet may as necessary contain conventional excipients such as binding agents, fillers, lubricants, glidants, disintegrants and wetting agents.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polyvinylpyrrolidone, polacrilin potassium, pregelatinised starch, sodium alginate, sodium lauryl sulphate, sodium starch glycollate.

An example of a pharmaceutically acceptable wetting agent is sodium lauryl sulphate.

As required the solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice.

Compositions may, if desired, be in the form of a pack accompanied by written or printed instructions for use.

No adverse toxicological effects are expected for the compositions of the invention in the above mentioned dosage ranges.

### EXAMPLES COMPRISING AN INSULIN SENSITISER AND A BIGUANIDE

### Example 1, Sustained release by use of a semi-permeable membrane

The semi-permeable membrane consists of:

| | %w/w |
|---|---|
| Eudragit RS30D (30% aqueous dispersion) | 90 |
| Triethyl Citrate | 1 |
| Talc | 9 |

This membrane is applied to a single or bilayer tablets each comprising 4mg or 8mg of Compound (I) and 500, preferably, or 1000 or 1500mg of metformin HCl

### Example 2, Sustained Release by use of a non disintegrating matrix tablet

A matrix tablet is formed by tabletting the following mixture as:
(a) a single layer tablet:

| | mg/tablet |
|---|---|
| Compound (I) | 4 (pfb) |
| Metformin HCl | 500 |
| Eudragit L100-55 | 150 |
| Lactose monohydrate | 50 |
| Eudragit RS powder to | 1000 |

(b) a bilayer tablet to provide sustained release of Compound I and immediate (i.e non-modified) release of metformin HCl.

| Layer A | mg/tablet |
|---|---|
| Compound (I) | 4 (pfb) |
| Eudragit L100-55 | 150 |
| Lactose monohydrate | 50 |
| Eudragit RS powder to | 500 |

| Layer B | mg/tablet |
|---|---|
| Metformin HCl | 500 |
| Polyvinyl pyrollidone | 15 |
| Magnesium stearate to | 520 |

### Example 3, Sustained Release by use of a Mixed Eudragit matrix tablet

A matrix tablet is formed by tabletting the following mixture as:
(a) a single layer tablet:

| | mg/tablet |
|---|---|
| Compound (I) | 4 (pfb) |
| Metformin HCl | 500 |
| Eudragit L100-55 | 74 |
| Eudragit RS powder | 18.5 |
| Colloidal Silicon dioxide | 2.6 |
| Magnesium stearate | 3.25 |
| Lactose monohydrate to | 650 |

(b) a trilayer tablet:

| Layer A | mg/tablet |
|---|---|
| Compound (I) | 4 (pfb) |
| Eudragit L100-55 | 74 |
| Eudragit RS powder | 18.5 |
| Colloidal Silicon dioxide | 0.6 |
| Magnesium stearate | 1.5 |
| Lactose monohydrate to | 150 |

| Layer B | mg/tablet |
|---|---|
| Metformin HCl | 250 |
| Eudragit L100-55 | 74 |
| Eudragit RS powder to | 345 |

| Layer C | mg/tablet |
|---|---|
| Metformin HCl | 250 |
| Polyvinyl pyrrolidone | 7.5 |
| Magnesium stearate to | 260 |

### Example 4, Sustained Release by use of a disintegrating matrix tablet

A matrix tablet is formed by tabletting the following mixture as a single layer tablet:

| | mg/tablet |
|---|---|
| Compound (I) | 4 (pfb) |
| Metformin HCl | 500 |
| Eudragit L100-55 | 74 |
| Methocel K4M | 18.5 |
| Colloidal Silicon dioxide | 2.6 |
| Magnesium stearate | 3.25 |
| Lactose monohydrate to | 650 |

### Example 5, Sustained Release by use of a Mixed Carbopol matrix tablet

A matrix tablet is formed by tabletting the following mixture as single or bilayer tablet:

| | mg/tablet |
|---|---|
| Compound (I) | 4 (pfb) |
| Metformin HCl | 500 |
| Anhydrous dibasic calcium phosphate | 35.7 |
| Carbopol 971P | 22.5 |
| Carbopol 974P | 7.5 |
| Talc | 0.75 |
| Lactose monohydrate to | 650 |

## Claims

1. A pharmaceutical composition suitable for once daily administration, which composition comprises:
(a) 2 mg to 12 mg of 5-[4-[2-(N-methyl-N-(2-pyridyl)amino)ethoxy]benzyl]thiazolidine-2,4-dione, optionally in the form of a pharmaceutically acceptable salt or solvate thereof;
(b) 100 mg to 3000 mg of metformin or a pharmaceutically acceptable salt thereof; and
(c) a pharmaceutically acceptable carrier;
wherein the composition is arranged to provide sustained release of 5-[4-[2-(N-methyl-N-(2-pyridyl)amino)ethoxy]benzyl]thiazolidine-2,4-dione and metformin, said sustained release being provided by a sustained release matrix selected from disintegrating, non-disintegrating and eroding matrices.

2. A composition according to claim 1, wherein the non-disintegrating matrix tablet formulation is provided by incorporating Eudragit RS, methacrylates, cellulose acetates, hydroxypropyl methylcellulose phthalate, Eudragit L, Carbopol 971P or HPMCP-HP-55S into the matrix.

3. A composition according to claim 1, wherein the disintegrating matrix tablet formulation is provided by incorporating methacrylates, methylcellulose and Methocel K4M into the matrix.

4. A composition according to any preceding claim, wherein 5-[4-[2-(N-methyl-N-(2-pyridyl)amino)ethoxy]benzyl]thiazolidine-2,4-dione is in the form of a maleate salt.

5. A composition according to any preceding claim, wherein metformin is in the form of a hydrochloride salt.

6. A process for preparing a pharmaceutical composition according to claim 1, which process comprises formulating [2-(N-methyl-N-(2-pyridyl)amino)ethoxy]benzyl]thiazolidine-2,4-dione or a pharmaceutically acceptable derivative thereof, metformin or a pharmaceutically acceptable salt thereof and the pharmaceutically acceptable carrier so as to enable sustained release of 5-[4-[2-(N-methyl-N-(2-pyridyl)amino)ethoxy]benzyl]thiazolidine-2,4-dione and metformin.

## Patentansprüche

1. Arzneimittel, geeignet zur einmal täglichen Verabreichung, wobei das Mittel umfasst:
(a) 2 mg bis 12 mg 5-[4-[2-(N-Methyl-N-(2-pyridyl)amino)ethoxy]benzyl]thiazolidin-2,4-dion, gegebenenfalls in der Form eines pharmazeutisch verträglichen Salzes oder Solvates davon;
(b) 100 mg bis 3000 mg Metformin oder ein pharmazeutisch verträgliches Salz davon; und
(c) einen pharmazeutisch verträglichen Träger umfasst;
wobei das Mittel zusammengestellt ist, um die verzögerte Freigabe von 5-[4-[2-(N-Methyl-N-(2-pyridyl)amino)ethoxy]benzyl]thiazolidin-2,4-dion und Metformin bereitzustellen, wobei die verzögerte Freigabe durch eine Matrix für verzögerte Freigabe bereitgestellt wird, ausgewählt aus zerfallenden, nicht-zerfallenden und erodierenden Matrices.

2. Mittel gemäß Anspruch 1, wobei die Tablettenrezeptur für eine nicht-zerfallende Matrix bereitgestellt wird durch Einbringen von Eudragit RS, Methacrylaten, Celluloseacetaten, Hydroxypropylmethylcellulosephthalat, Eudragit L, Carbopol 971P oder HPMCP-HP-55S in die Matrix.

3. Mittel gemäß Anspruch 1, wobei die Tablettenrezeptur für eine zerfallende Matrix bereitgestellt wird durch Einbringen von Methacrylaten, Methylcellulose und Methocel K4M in die Matrix.

4. Mittel gemäß einem der vorstehenden Ansprüche, wobei 5-[4-[2-(N-Methyl-N-(2-pyridyl)amino)ethoxy]benzyl]thiazolidin-2,4-dion in der Form eines Maleatsalzes vorliegt.

5. Mittel gemäß einem der vorstehenden Ansprüche, wobei das Metformin in der Form eines Hydrochloridsalzes vorliegt.

6. Verfahren zur Herstellung eines Arzneimittels gemäß Anspruch 1, wobei das Verfahren das Formulieren von 5-[4-[2-(N-Methyl-N-(2-pyridyl)amino)-ethoxy]benzyl]thiazolidin-2,4-dion oder eines pharmazeutisch verträglichen Derivats davon, Metformin oder eines pharmazeutisch verträglichen Salzes davon und des pharmazeutisch verträglichen Trägers umfasst, um die verzögerte Freigabe von 5-[4-[2-(N-Methyl-N-(2-pyridyl)amino)ethoxy]benzyl]thiazolidin-2,4-dion und Metformin zu ermöglichen.

## Revendications

1. Composition pharmaceutique convenable pour l'administration une fois par jour, composition qui comprend :
(a) 2 mg à 12 mg de 5-[4-[2-(N-méthyl-N-(2-pyridyl)-amino)éthoxy]benzyl]thiazolidine-2,4-dione, éventuellement sous forme d'un de ses sels ou produits de solvatation pharmaceutiquement acceptables ;
(b) 100 mg à 3000 mg de metformine ou d'un de ses sels pharmaceutiquement acceptables ; et
(c) un support pharmaceutiquement acceptable ;
ladite composition étant conçue pour provoquer la libération prolongée de la 5-[4-[2-(N-méthyl-N-(2-pyridyl)-amino)éthoxy]benzyl]thiazolidine-2,4-dione et de la metformine, ladite libération prolongée étant provoquée par une matrice à libération prolongée choisie entre des matrices à délitement, des matrices sans délitement et des matrices à érosion.

2. Composition suivant la revendication 1, dans laquelle la formulation de comprimé à matrice sans délitement est fournie en incorporant de l'Eudragit RS, des méthacrylates, des acétates de cellulose, du phtalate d'hydroxypropylméthylcellulose, de l'Eudragit L, du Carborpol 971P ou du HPMCP-HP-55S à la matrice.

3. Composition suivant la revendication 1, dans laquelle la formulation de comprimé à matrice à délitement est fournie en incorporant des méthacrylates, de la méthylcellulose et du Methocel K4M à la matrice.

4. Composition suivant l'une quelconque des revendications précédentes, dans laquelle la 5-[4-[2-(N-méthyl-N-(2-pyridyl)-amino)éthoxy]benzyl]thiazolidine-2,4-dione est sous forme d'un sel maléate.

5. Composition suivant l'une quelconque des revendications précédentes, dans laquelle la metformine est sous forme d'un sel chlorhydrate.

6. Procédé pour la préparation d'une composition pharmaceutique suivant la revendication 1, procédé qui comprend la formulation de [2-(N-méthyl-N-(2-pyridyl)-amino)éthoxy]benzyl]thiazolidine-2,4-dione ou d'un de ses dérivés pharmaceutiquement acceptables, de metformine ou d'un de ses sels pharmaceutiquement acceptables et du support pharmaceutiquement acceptable de manière à permettre la libération prolongée de la 5-[4-[2-(N-méthyl-N-(2-pyridyl)-amino)éthoxy]benzyl]thiazolidine-2,4-dione et de la metformine.
